# EUROPÄISCHE PATENTSCHRIFT

(11) **EP 0 796 598 B1**
(45) Veröffentlichungstag und Bekanntmachung des Hinweises auf die Patenterteilung: **10.07.2002**
(21) Anmeldenummer: 97103833.6
(22) Anmeldetag: 07.03.1997
(51) Int. Cl.: A61F 2/34, A61F 2/46

(54) **Prothetische Gelenkpfanne**
Prosthetic joint cup
Cotyle prothétique

(30) Priorität: 22.03.1996 DE 19611248
(43) Veröffentlichungstag der Anmeldung: 24.09.1997
(73) Patentinhaber: CERASIV GmbH INNOVATIVES KERAMIK-ENGINEERING, D-73207 Plochingen (DE)
(72) Erfinder: Pfaff, Hans-Georg, 73760 Ostfildern (DE); Küsswetter, W., Prof. Dr., 72076 Tübingen (DE)
(74) Vertreter: Scherzberg, Andreas, Dr.

(56) Entgegenhaltungen:
- EP-A- 0 649 641
- EP-A- 0 694 294
- DE-U- 29 703 857

## Beschreibung

Die Erfindung bezieht sich auf eine prothetische Gelenkpfanne mit einer Außenschale, in der eine austauschbare Einsatzschale eingesetzt ist.

Insbesondere bei Hüftgelenken sind die Einsatzschalen, die eine Gelenkkugel aufnehmen, einem hohen Verschleiß ausgesetzt. Deshalb muß zu gegebenem Zeitpunkt die abgenutzte Einsatzschale in situ (bei implantierter Gelenkpfanne) ausgetauscht werden. Die Einsatzschale muß zur Entfernung aus der Außenschale beschädigt oder zerstört werden. Dabei können hohe mechanische Belastungen auftreten und Splitter des Einsatzschalenmaterials in das umliegende Gewebe geraten.

Bei der prothetischen Gelenkpfanne gemäß Dokument EP-A-0649641, die den Oberbegriff des Anspruchs 1 bildet, sind in der Außenschale zwei Einkerbungen vorgesehen, die ein Heraushebeln der Einsatzschale mittels geeigneter Werkzeuge erleichtern sollen.

Aufgabe der Erfindung ist es, eine prothetische Gelenkpfanne mit einer Einsatzschale zu schaffen, deren Entfernbarkeit verbessert ist.

Die Aufgabe wird dadurch gelöst, daß die Einsatzschale einen Außenkonus-Abschnitt aufweist, der in einen Innenkonus-Abschnitt der Außenschale eingepaßt ist, so daß zwischen den Konusabschnitten der Einsatzschale und der Außenschale eine haltende Klemmkraft auftritt. Durch diese Klemmbefestigung ist einerseits ein fester Sitz der Einsatzschale in der Außenschale gewährleistet, andererseits jedoch auch die Lösbarkeit dieser Befestigung sichergestellt. Die Außenschale weist an ihrem Öffnungsrand mindestens einen Ansatz auf; an den eine Schlagvorrichtung unverschiebbar ansetzbar ist. Zum Lösen des Klemmsitzes wird mit der Schlagvorrichtung mindestens ein mechanischer Impuls auf den Öffnungsrand übertragen, wodurch der Klemmsitz zwischen Einsatzschale und Außenschale gelöst und die Einsatzschale aus der Außenschale herausgetrieben wird. Die Schlagvorrichtung wird an den Ansatz angesetzt, so daß der mechanische Impuls an einem definierten Ort in die Außenschale eingeleitet wird und die Schlagvorrichtung nicht von dem Öffnungsrand abgleiten und das umliegende Gewebe schädigen kann.

Der Konuswinkel der Konusabschnitte der Außenschale und der Einsatzschale ist gleich und beträgt zwischen 14 und 22°. In diesem Konuswinkelbereich ist einerseits eine ausreichende Klemmwirkung zwischen Außenschale und Einsatzschale gewährleistet, so daß eine ungewollte Lockerung der Einsatzschale ausgeschlossen ist. Andererseits ist jedoch die Selbsthemmung so gering, daß ein problemloses Lösen des Klemmsitzes durch die Schlagvorrichtung möglich ist. Die Einsatzschale kann auf diese Weise schnell und mit einfachen Mitteln in situ entfernt werden, ohne daß die Einsatzschale beschädigt oder zerstört werden muß. Das Entfernen der

Einsatzschale ist außerdem splitterfrei, so daß keine entsprechenden Schutzmaßnahmen getroffen werden müssen.

Der Bodenbereich der Außenschale und der Bodenbereich der Einsatzschale können einen Hohlraum bilden. Durch den Hohlraum wird verhindert, daß die Einsatzschale in ihrem Bodenbereich die Außenschale berührt, bevor ein ausreichender Klemmsitz zwischen den Konus-Abschnitten der eingesetzten Einsatzschale und der Außenschale erreicht ist.

Der Ansatz auf dem öffnungsrand ist vorzugsweise eine muldenartige Ausnehmung, in die eine Ausstülpung der Schlagvorrichtung einsetzbar ist. Dadurch ist mit einfachen Mitteln ein Ansatz geschaffen, der die Schlagvorrichtung zuverlässig auf dem Öffnungsrand fixiert und ein Abgleiten der Schlagvorrichtung ausschließt.

Die Außenschale kann aus Metall und die Innenschale aus Keramik bestehen. Keramik ist wegen seiner Härte, seines geringen Abriebes, langen Haltbarkeit und guten Verträglichkeit für die Innenschale besonders geeignet.

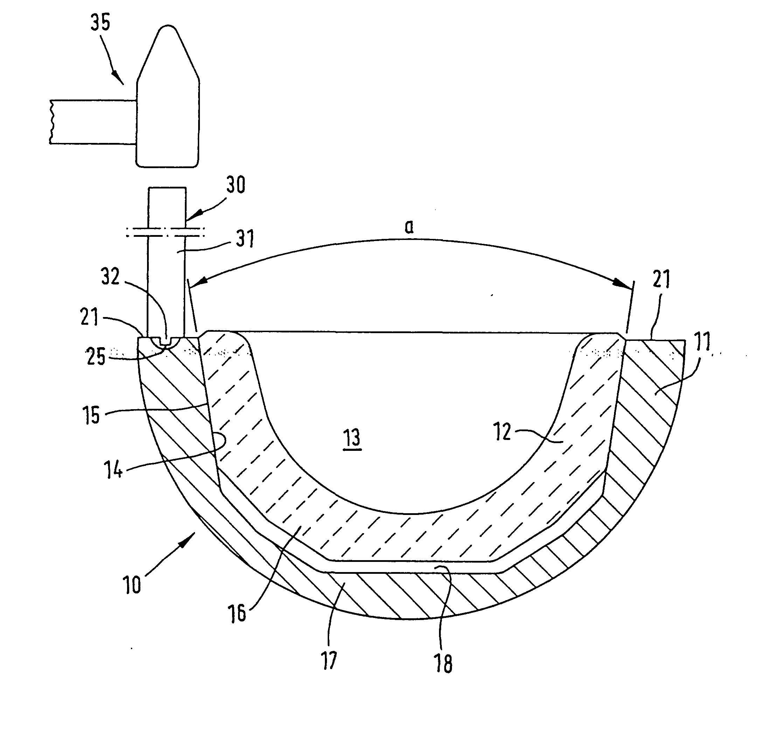

Im folgenden wird unter Bezugnahme auf die Figur ein Ausführungsbeispiel der Erfindung näher erläutert.

Die Figur zeigt einen Querschnitt einer prothetischen Gelenkpfanne mit einer angesetzten Schlagvorrichtung.

Eine prothetische Gelenkpfanne 10 wird von einer Außenschale 11 und einer darin eingesetzten Einsatzschale 12 gebildet. Die Gelenkpfanne 10 dient der Aufnahme einer prothetischen Gelenkkugel (nicht dargestellt).

Die Außenseite der Außenschale 11 ist kugelförmig ausgebildet, kann jedoch auch andere Formen haben und dem jeweiligen Zweck und Implantationsort angepaßt sein. Sie besteht aus Metall, kann jedoch auch aus anderen Werkstoffen bestehen.

An der Innenseite weist die Außenschale 11 an den öffnungsrand angrenzend einen Innenkonus mit einem Konuswinkel a von 20° auf. Der Innenkonus-Abschnitt 14 erstreckt sich über ungefähr die halbe Höhe der Außenschale 11. Im Bodenbereich der Außenschale 11 schließt sich an den Innenkonus-Abschnitt 14 die Bodenwand 17 an.

Die Einsatzschale 12 weist eine sphärische Ausnehmung 13 zur Aufnahme der Gelenkkugel auf. An der Außenseite weist sie einen Außenkonus-Abschnitt 15 mit dem gleichen Konuswinkel a auf, wie der Innenkonus 14 der Außenschale 11. Die wirksame Länge des Außenkonus 15 ist etwas kürzer als die des Innenkonus 14. An den Außenkonus-Abschnitt 15 schließt sich im Bodenbereich der Einsatzschale 12 eine Bodenwand 16 an, die bei eingesetzter Einsatzschale 12 nicht die Bodenwand 17 der Außenschale 11 berührt. Die Bodenwände 16,17 in der Einsatzschale 12 und der Außenschale 11 bilden einen Hohlraum 18, der ein Aufsetzen der Einsatzschale 12 in der Außenschale 11 ausschließt.

Der Öffnungsrand der Außenschale 11 ist als eine schmale Ringfläche 21 ausgebildet, die senkrecht zur Längsachse der Gelenkpfanne 10 angeordnet ist. Auf der Ringfläche 21 ist als Ansatz für eine Schlagvorrichtung 30 eine Ausnehmung 25 vorgesehen. Die Ringfläche 21 kann auch mehrere Ausnehmungen enthalten, um den Schlagkörper stets an einer leicht zugänglichen Ausnehmung ansetzen zu können.

Als Schlagvorrichtung 30 wird ein meißelartiger Schlagkörper 31 verwendet, der an einem Ende eine Ausstülpung 32 aufweist, die in die Ausnehmung 25 paßt. Zur Erzeugung von Schlagimpulsen wird ein Hammer 35 verwendet, mit dem auf das freie Ende der Schlagvorrichtung 30 geschlagen wird.

Die Einsatzschale 12 besteht aus Keramik und die Außenschale 11 aus Metall, die Schalen können jedoch jeweils auch aus anderen Materialien bestehen.

Während ihres prothetischen Einsatzes ist die Innenfläche der Ausnehmung 13 der Einsatzschale 12 ständigem Verschleiß ausgesetzt. Insbesondere bei den stark beanspruchten Hüftgelenken kann daher der Austausch der verschlissenen Einsatzschale 12 früher oder später erforderlich sein. Dieser Austausch wird in situ vorgenommen, d.h. die Gelenkpfannen-Prothese verbleibt dabei in dem jeweiligen Knochen.

Nach dem Zugänglichmachen des Gelenks wird die Gelenkkugel (nicht gezeigt) aus der Gelenkpfanne 10 entnommen, so daß die Gelenkpfanne 10 von ihrer Öffnungsseite her frei zugänglich ist. Nun wird der Schlagkörper 31 mit seiner stirnseitigen Ausstülpung 32 in die Ausnehmung 25 auf der Ringfläche 21 eingesetzt.

Wenn der Schlagkörper 31 sicher auf der Ringfläche 21 sitzt, wird mit dem Hammer 35 vorsichtig mindestens ein Schlag über den Schlagkörper 31 auf die Ringfläche 21 der Außenschale 11 aufgebracht. Durch die kurze Beschleunigung und die Verformung der Außenschale 11 löst sich die Einsatzschale 12 aus ihrem Klemmsitz in der Außenschale 11 und kann leicht entnommen werden. Anschließend kann eine neue Einsatzschale 12 in die Außenschale mit einem geeigneten Eindrückwerkzeug eingesetzt und mit Klemmsitz in der Außenschale 11 befestigt werden.

Das Entfernen und Austauschen der Einsatzschale ist einfach, schnell und splitterfrei vornehmbar.

## Patentansprüche

1. Prothetische Gelenkpfanne mit einer Außenschale (11), in der mit Klemmsitz eine austauschbare Einsatzschale (12) eingesetzt ist, wobei
die Einsatzschale (12) einen Außenkonus-Abschnitt (15) aufweist, der in einen Innenkonus-Abschnitt (14) der Außenschale (11) eingepaßt ist, so daß zwischen den Abschnitten (14,15) der Außenschale (11) und der eingesetzten Einsatzschale (12) eine haltende Klemmkraft auftritt,
**dadurch gekennzeichnet, daß**
die Außenschale (11) an ihrem Öffnungsrand (21) mindestens einen Ansatz (25) aufweist, an den eine Schlagvorrichtung (30) unverschiebbar ansetzbar ist.

2. Prothetische Gelenkpfanne nach Anspruch 1, **dadurch gekennzeichnet, daß** der Konuswinkel (a) der Konusabschnitte (14,15) der Außenschale (11) und der Einsatzschale (12) gleich ist und zwischen 14° und 22° beträgt.

3. Prothetische Gelenkpfanne nach Anspruch 1 oder 2, **dadurch gekennzeichnet, daß** der Bodenbereich (17) der Außenschale (11) und der Bodenbereich (16) der Einsatzschale (12) einen Hohlraum (18) bilden.

4. Prothetische Gelenkpfanne nach einem der Ansprüche 1-3, **dadurch gekennzeichnet, daß** der Ansatz (25) eine muldenartige Ausnehmung ist, in die eine Ausstülpung (32) der Schlagvorrichtung (30) einsetzbar ist.

5. Prothetische Gelenkpfanne nach einem der Ansprüche 1 bis 5, **dadurch gekennzeichnet, daß** die Außenschale (11) aus Metall und die Innenschale (12) aus Keramik besteht.

## Claims

1. Prosthetic joint socket with an outer shell (11), in which a replaceable insert shell (12) is inserted with a clamping fit, the insert shell (12) having an outer-cone section (15) which is fitted into an inner-cone section (14) of the outer shell (11), so that a retaining clamping force occurs between the sections (14,15) of the outer shell (11) and of the inserted insert shell (12), **characterised in that** the outer shell (11) has, at the edge (21) of its opening, at least one area of application (25), to which a striking device (30) can be non-relocatably applied.

2. Prosthetic joint socket according to Claim 1, **characterised in that** the cone angle (a) of the cone sections (14,15) of the outer shell (11) and of the insert shell (12) are equal and are between 14° and 22°.

3. Prosthetic joint socket according to Claim 1 or 2, **characterised in that** the bottom region (17) of the outer shell (11) and the bottom region (16) of the insert shell (12) form a hollow space (18).

4. Prosthetic joint socket according to one of Claims 1-3, **characterised in that** the area of application (25) is a trough-like recess, into which a protrusion (32) of the striking device (30) can be inserted.

5. Prosthetic joint socket according to one of Claims 1 to 5, **characterised in that** the outer shell (11) consists of metal and the inner shell (12) consists of ceramic.

## Revendications

1. Cotyle prothétique comprenant une coque (11) extérieure dans laquelle un insert (12) intérieur remplaçable est tenu par serrage, l'insert (12) intérieur comportant une partie (15) en forme de cône extérieur qui est ajustée dans une partie (14) en forme de cône intérieur de la coque (11) extérieure, de manière à obtenir entre les parties (14, 15) de la coque (11) extérieure et de l'insert (12) intérieur monté dans celle-ci une force de serrage durable, **caractérisé en ce que** la coque (11) extérieure, au niveau du bord (21) de son ouverture, comporte au moins une embase (25), sur laquelle un dispositif à choc (30) peut être appliqué sans risque de glissement.

2. Cotyle prothétique selon la revendication 1, **caractérisé en ce que** l'angle de cône (a) des parties (14, 15) en forme de cône de la coque (11) extérieure et de l'insert (12) est identique et est compris entre 14° et 22°.

3. Cotyle prothétique selon la revendication 1 ou 2, **caractérisé en ce que** la portion de fond (17) de la coque (11) extérieure et la portion de fond (16) de l'insert (12) intérieur définissent un vide (18).

4. Cotyle prothétique selon une des revendications 1 à 3, **caractérisé en ce que** l'embase (25) est une dépression en forme de cuvette dans lequel un renflement (32) du dispositif à choc (30) peut être inséré.

5. Cotyle prothétique selon une des revendications 1 à 4, **caractérisé en ce que** la coque (11) extérieure est en métal et l'insert (12) intérieur en céramique.
